# EUROPEAN PATENT APPLICATION

(11) **EP 2 175 020 A1**
(43) Date of publication of application: **14.04.2010**
(21) Application number: 08017905.4
(22) Date of filing: 13.10.2008
(51) Int. Cl.: C12N 15/10

(54) **Reduction of RNase activity in complex fluidic samples**

(71) Applicant: Roche Diagnostics GmbH, 68298 Mannheim (DE); F. Hoffmann-La Roche AG, 4070 Basel (CH)
(72) Inventor: Adie, Sigrid, 94099 Ruhstorf (DE); Kraiss, Stefan, 82515 Wolfratshausen (DE); Pasche, Jan Paul, 6005 Luzern (CH); Russmann, Eberhard, 92377 Penzberg (DE); Walter, Thomas, 92377 Penzberg (DE)

(57) **Abstract**

The invention provides compositions and methods for reducing RNase activity, thereby stabilizing RNA molecules in complex samples. A sample or a lysate thereof is combined and incubated with a compound capable of reacting in aqueous solution with a functional group of an amino acid residue in a polypeptide, whereby the functional group is selected from the group consisting of a primary amino group and a thiol group, whereby in the composition said compound is present at a concentration of between about 0.1 M and about 1 M.

## Description

The present patent application is directed to the inhibition of RNase enzymatic activities in fluidic samples. The invention provides compositions and methods for reducing RNase activity, thereby stabilizing RNA molecules in complex samples. A sample or a lysate thereof is combined and incubated with a compound capable of reacting in aqueous solution with a functional group of an amino acid residue in a polypeptide, whereby the functional group is selected from the group consisting of a primary amino group and a thiol group, whereby in the composition said compound is present at a concentration of between about 0.1 M and about 1 M.Background of the Invention

RNA (ribonucleic acid) is the least stable of the biopolymers that effect information transfer in biology. To the largest part this is due to degradation by ribonucleases. Ribonuclease, abbreviated commonly as RNase, generically denotes a nuclease that catalyzes the breakdown of RNA into smaller components. For the understanding of the present invention, a RNase is a nuclease specifically catalyzing the cleavage of phosphate ester linkages in RNA molecules. Such catalysis is also referred to as RNase (enzymatic) activity.

Because RNases play an important role in nucleic acid metabolism, they are found in both prokaryotes and eukaryotes, and in practically every cell type. The human body uses RNases to defend against invading microorganisms by secreting these enzymes in fluids such as tears, saliva, mucus and perspiration. The primary source of RNases within most environments, however, is microorganisms - namely bacteria, fungi and their spores. In a laboratory setting, RNase contamination problems are exacerbated since microorganisms and their derivatives (e.g., restriction enzymes, polymerases, cell lysis promoting proteinases, etc.) are frequently used as essential tools or may even be the object of scientific study. Particularly, RNase activity poses a serious problem in the technical field of RNA preparation from biological samples. The ubiquitous presence of RNase enzymatic activity usually results in very short lifespans for any RNA that is not in a protected environment.

RNases, especially those belonging to the RNase A family, are fairly small, compact proteins of about 13.7 kDa containing several cysteine residues that form numerous intramolecular disulfide bonds. As a result, denatured RNases tend to regain their native structure and partial function after being cooled to room temperature in the absence of a denaturant. Consequently, RNases can retain activity after freeze-thaw cycles and even autoclaving. The robust nature of these enzymes makes them refractory to many methods of decontamination. Indeed, drastic chemical methods are often required to inactivate RNase activity on surfaces and in solutions, or to remove the RNases responsible for such activity.

Known methods of RNase inactivation in biological sample matter can be grouped with regard to an either a specific or a generic mode of action. The specific mode includes the interaction of RNases with proteinaceous RNase inhibitors, e.g. commonly available under the trade name RNASIN®. The 50kDa RNase inhibitor exerts its inhibitory effect by noncovalently binding to RNases in a 1:1 ratio and with an extraordinary affinity. The Kᵢ value for binding of RNASIN® ribonuclease inhibitor to RNase A is approximately 10⁻¹⁴ M. In addition, certain molecules of low molecular weight such as vanadyl ribonucleoside are known to have a high affinity for RNases and to form complexes, thereby inactivating RNase enzymatic activity. Adding reducing agents such as dithiothreitol (DTT) can enhance the inhibitory effect. However, specific inactivation of RNases does not inhibit the members of all different families of RNase enzymes equally well. Thus, depending on the origin of the biological sample and the compatibility of customary proteinacious RNase inhibitors to the RNases present in that sample, RNA purified therefrom may not be sufficiently protected from enzymatic degradation.

In contrast, the generic mode of action targets not only RNases but proteins in general. Numerous procedures of sample preparation for RNA purification include denaturing agents and treatment with proteases. Proteolysis abolishes RNase enzymatic activity if the RNase enzymes in the sample are sufficiently degraded to inactive peptides. For example, EP 1229134 discloses a combination of Proteinase K digestion and phenol-chloroform extraction to remove contaminating RNases of a sample comprising a cell-free extract.

Degradation can also require the presence of denaturing agents such as sodium dodecyl sulfate (SDS). However, since biological samples frequently contain a large quantity of other proteins, degradation of RNases may be incomplete due to competition effects. In addition, even certain RNase fragments are known to retain some RNase enzymatic activity.

There are also procedures for the preparation of biological samples making use of guanidine thiocyanate or other chaotropic salts, in order to lyse the sample matter and at the same time inhibit RNases. Particularly guanidine thiocyanate is known to be a potent RNase inhibitor when applied at high concentrations, e.g. higher than about 2M. Furthermore. Other procedures use acidic phenol as a lysis reagent to achieve the same effect. However, in order to inactivate RNase enzymatic activity unspecifically the inactivating reagents usually have to be added in high quantities for sufficient efficacy. Thus, solutions containing these reagents occupy large volumes in relation to the sample volume. However, procedures for sample preparation are desired in which the volume of the processed sample is as small as possible. Low volumes provided, loss of RNA due to dilution of the processed sample can be avoided more easily.

Apart from the biological sample matter, buffers and solutions are a particular source of RNase contamination. Thus, it is common practice to prepare RNase-free solutions. But merely autoclaving prepared solutions is not sufficient for eliminating RNase contamination. Solutions need to be treated with RNase inactivation reagents of which diethylpyrocarbonate (DEPC) is one of the most commonly known. DEPC reacts with the ε-amino groups of lysine and the carboxylic groups of aspartate and glutamate both intra-and intermolecularly (Wolf et al., Eur. J. Biochem. 13 (1970) 519-525). This chemical reaction forms polymers of the ribonuclease.

In order to inactivate RNase activity in a solution, the treatment typically involves incubating said solution with 0.1% DEPC for one or more hours, usually overnight, at a temperature between about room temperature and about 37°C. Incubation is followed by autoclaving the solution to eliminate residual DEPC. Inactivation of DEPC in this manner yields CO₂, H₂O and ethanol. DEPC is not used with Tris buffer or HEPES buffer since these buffer salts inactivate DEPC by reacting with it.

An alternative to DEPC is the RNase inactivation reagent dimethyl dicarbonate (DMDC). DMDC is also known by the trade name VELCORIN®. This reagent is also known to modify primary -NH groups of proteins.

Mendelsohn SL & Young DA Biochim. Biophys. Acta 519 (1978) 461-473) have studied the effectiveness of RNase inhibitors in an RNA isolation protocol. DEPC alone or DEPC and SDS were added to cytoplasmic fractions of thymus cells, and RNA was isolated using phenol extraction. DEPC at a concentration of 0.5% and in the presence of SDS was concluded to be most effective under these conditions.

US 7,148,343 discloses methods of RNA isolation in which RNA is adsorbed to a solid support from a liquid mixture containing lysed sample material, whereby adsorption occured in the presence of lithium ions. Prior to elution of the RNA the the solid support was washed with DEPC water. The buffer used for elution of the RNA from the support was also treated with DEPC.

The technical problem underlying the present invention was to provide an alternative method of RNase inactivation in biological sample material which is to be processed for RNA purification. Thus, an object of the invention is to provide a method for the purification of RNA from a biological sample, whereby the RNA is stabilized.

The inventors surprisingly found that RNases in biological sample material can be rapidly and completely inactivated by adding to the sample material a reagent which is capable of modifying primary amino groups. Even low amounts of the reagent are sufficient to reach the desired effect.

### Summary of the Invention

A first aspect of the invention is a composition consisting of (i) a liquid sample comprising RNA and protein with RNase activity, and (ii) a compound capable of reacting in aqueous solution with a functional group of an amino acid residue in a polypeptide, whereby the functional group is selected from the group consisting of a primary amino group and a thiol group, **characterized in that** in the composition said compound is present at a concentration of between about 0.5 M and about 1 M. Another aspect of the invention is a method to reduce RNase activity in a liquid sample comprising RNA and protein with RNase activity, comprising the step of forming a composition according to the invention, and incubating said composition. A further aspect of the invention is a method to isolate RNA from a liquid sample comprising RNA and protein with RNase activity, comprising the steps of (a) reducing RNase activity in said sample by applying the method according to any of the claims 7 to 9, whereby a composition with reduced RNase activity is provided; followed by (b) purifying the RNA from said composition. Yet, a further aspect of the invention is a method for determining the presence of a target RNA molecule in a liquid sample comprising RNA and protein with RNase activity, comprising the steps of (a) isolating RNA from the sample according to any of the claims 10 and 11; followed by (b) detecting in the isolated RNA the presence of the target RNA molecule. Yet, a further aspect of the invention is a kit of parts, comprising in separate containers (i) a compound capable of reacting in aqueous solution with a functional group of an amino acid residue in a polypeptide, whereby the functional group is selected from the group consisting of a primary amino group and a thiol group; (ii) a solid phase capable of reversibly binding RNA; (iii) a reagent for effecting lysis of biological material.

### Detailed Description of the Invention

Certain terms are used with particular meaning, or are defined for the first time, in this description of the present invention. For the purposes of the present invention, the terms used are defined by their art-accepted definitions, when such exist, except that when those definitions conflict or partially conflict with the definitions set forth below. In the event of a conflict in definition, the meaning of a terms is first defined by any of the definitions set forth below.

The term "comprising" is used in the description of the invention and in the claims to mean "including, but not necessarily limited to". In contrast, the term "consisting of" followed by a list of items is used to mean "being composed of the items of the list and excluding other items which are not listed".

The articles "a" and "an" are used herein to refer to one or to more than one (i.e. to at least one) of the grammatical object of the article. By way of example, "a compound" means one compound or more than one compound.

When designating a range of numerical values such as a concentration range, the range is indicated by the word "between", followed by a first value n1 and a second value n2. The lower boundary of the designated range is understood as being the value equal to or higher than the first value. The higher boundary of the designated range is understood as being the value equal to or lower than the second value". Thus, a value x in the designated range is given by n1 ≤ x ≤ n2.

If not stated otherwise, it is understood that the term "about" and the character "∼" in combination with a numerical value n ("about n", "∼n") indicates a value x in the interval given by the numerical value ±5% of the value, i.e. n - 0.05 * n ≤ x ≤ n + 0.05 * n. In case the term "about" or the character "∼" in combination with a numerical value n describes a preferred embodiment of the invention, the value of n is most preferred, if not indicated otherwise.

It was a surprising finding by the inventors that in a fluidic biological sample, particularly in a sample with a high protein content (such as plasma or serum), RNase can be rapidly reduced or even inactivated by adding a compound capable of modifying primary amino groups, and reacting the sample material therewith.

Amino groups in proteins are provided by the terminal amine if this has not been derivatised, and by the epsilon-amino groups of lysine residues which is very reactive. The epsilon-amino group (i.e. four carbon atoms distant from that containing the acid function) is highly reactive and the (-CH₂-)₄ chain acts as a convenient spacer to distance the reaction site from other moieties of the protein molecule. Amino groups are electron rich and nucleophilic. Shielded by the alkyl chain from any deactivating influences, the epsilon-amino group in lysine behaves as a typical amine; it is a strong base with a pKₐ in proteins of 9-9.5. Therefore it is protonated and unreactive under nonbasic and amine reactions are typically carried out in the pH range 7-9 which is preferred for practicing the present invention. More preferred, protein amino groups are reacted with the compound capable of reacting in aqueous solution with a functional group of an amino acid residue in a polypeptide at a neutral or weakly alkaline pH between about 7 and about 8.5, even more preferred at a pH at about 7.5. More alkaline conditions would favour amine reactivity but are usually discounted to avoid any damage to RNA.

Similar results concerning RNase inactivation were obtained with a compound capable of modifying thiol groups such as those present in cysteine residues. Thiol or mercaptan (SH) groups have unpaired electrons which make them strong Lewis bases, giving them nucleophilic and reducing properties This reactivity means that cysteine is most commonly found the form of dimeric cystine moieties. However, cysteine is sometimes encountered in its free state, and is a valuable target for chemical modification. The thiolate anion, which exists predominantly above the relevant pKₐ (approximately 8.5 in most proteins, but much lower in others), is much more nucleophilic than the neutral thiol. Thiols are soft bases, unlike amines which are hard, and therefore they do not react well with hard acids such as COOH groups and their derivatives. So while like amines they are strong nucleophiles, thiols can be used differently in protein derivatisation. To this end, reactions with the iodoacetate anion and maleimides can be used to modify RNase proteins, in order to reduce or even abolish their activity.

According to the invention, the modifying compound is applied at concentrations which are about 10 times higher than the concentrations known to be used for removal of RNase activity from buffer solutions to be used for RNA biochemistry. For example, the standard concentration of DEPC or DMDC applied to aqueous buffers is 0.1% [v/v]. Remarkably and unforeseen, about the same concentration as well as higher concentrations efficiently reduce and may even eliminate RNase activity in sample material with a high protein content such as plasma and serum. Such samples are complex mixtures containing a large number of proteins and other substances, many of which are possible targets for chemical modification of amino and thiol groups.

According to the invention, the compounds found by the inventors to be effective for RNase inhibition are used to form compositions consisting of sample material and the compound, whereby the compound is applied at a concentration of between about 0.1 M and about 1 M. Higher concentrations of the compound are possible but may cause unwanted side effects such as extensive crosslinking of proteins in the sample.

The term "sample" (or "sample material") as used herein refers to a liquid complex mixture comprising RNA molecules and proteins, whereby the proteins comprise one or more enzymes with RNase activity. According to the invention, the content of dissolved protein in the sample material is preferably between about 5 mg/ml and about 150 mg/ml. More preferred, the protein content is between about 20 mg/ml and about 120 mg/ml, even more preferred between about 50 mg/ml and about 100 mg/ml, most preferred about 70 mg/ml.

The sample is not necessarily a solution, it can comprise particulate matter, too. More preferred, the complex mixture is a biological sample, that is to say material comprising biological matter. The complex mixture may further contain a plurality of organic and inorganic compounds. In the context of the present invention it is desired to separate RNA molecules present in the sample from the proteins and other organic and inorganic compounds. The term "sample" encompasses an aqueous solution containing RNA derived from other origins, e.g. from chemical or enzymatic reaction mixtures, or from a previous purification of biological sample material. The term biological sample, from which RNA is purified, also encompasses samples comprising viruses or bacterial cells, as well as isolated cells from multicellular organisms such as human and animal cells as well as tissues and cell cultures. Particularly, the sample can contain leucocytes, and other immunologically active cells, chemical compounds with a low and/ or a high molecular weight such as haptens, antigens, antibodies and nucleic acids. The sample can be whole blood, blood serum, blood plasma, cerebral fluid, sputum, stool suspension, biopsy specimens, bone marrow, oral rinses, tissues, urine, or mixtures thereof. The present invention particularly encompasses biological samples such as a fluid from the human or animal body; very much preferred, the biological sample is blood, blood plasma, blood serum or urine. The blood plasma is preferably EDTA, heparin or citrate blood plasma. In an embodiment of the invention the biological sample comprises bacterial cells, eukaryotic cells, viruses or mixtures thereof. A biological sample as exemplified above, preferably in a processed form such as a lysate, can be part of the composition from which the (target) nucleic acid is adsorbed to the substrate. Also encompassed by the term "biological sample" are cells from plants, and fungi as well as other single cell organisms.

When preparing a composition of the invention, the sample material is used either untreated or as a lysate. Untreated sample material is preferred over lysates in the case that the sample does not contain particulate matter such as cells or cell debris. Th the latter case the preferred liquid sample according to the invention is a lysate. A "lysate" or a "lysed sample" can be obtained from a complex sample and/ or biological sample material comprising tissue, cells, bacteria or viruses, whereby the structural integrity of the material is disrupted. To release the contents of cells, tissue or, more generally, from the particles which make up a biological sample, the material is treated with enzymes or with chemicals to dissolve, degrade or denature the cellular walls and cellular membranes of such organisms. This process is encompassed by the term "lysis".

Preferred enzymes and enzyme mixtures to degrade cellular walls include lysozyme, lyticase, cellase, and chitinase. Preferred proteolytic enzymes include pronase, proteinase K, esperase, and collagenase.

In line with the present invention, one can further lyse particulate and/or cellular material of the sample using chaotropic agents such as a guanidine salt and/ or anionic, cationic, zwitterionic or non-ionic detergent. According to the invention, a detergent which is substantially inert towards (i.e. does not react with) the reactive compound capable of modifying primary amino groups and thiol groups is chosen.

By way of the lysis process, RNA is set free from complexing agents of high molecular weight such as proteins. It is also an advantage to use proteases which degrade an amount of the enzymes with nucleolytic activity and other unwanted proteins. In case there remains particulate, i.e. undissolved matter of the sample material following the lysis process, the particulate matter is usually separated from the lysate to result in a cleared lysate. This can be done, e.g., by way of filtering or centrifugation. In such a case the cleared lysate is processed further, e.g. by a method according to the invention. Thus, the term "lysed sample" encompasses a cleared lysate.

Thus, a first aspect of the invention is a composition consisting of (i) a liquid sample comprising RNA and protein with RNase activity, and (ii) a compound capable of reacting in aqueous solution with a functional group of an amino acid residue in a polypeptide, whereby the functional group is selected from the group consisting of a primary amino group and a thiol group, **characterized in that** in the composition said compound is present at a concentration of between about 0.1 M and about 1 M. A further aspect of the invention is the use of a compound capable of reacting in aqueous solution with a functional group of an amino acid residue in a polypeptide, whereby the functional group is selected from the group consisting of a primary amino group and a thiol group, for reducing RNase activity in a liquid sample comprising RNA and protein with RNase activity, whereby in the composition said compound is present at a concentration of between about 0.1 M and about 1 M.

In a preferred embodiment of the invention, the compound is selected from the group consisting of a pyrocarbonic acid ester, a salt with an iodoacetate anion, and maleimide or a maleimide derivative. The term "pyrocarbonic acid ester" denotes an ester of the hypothetical pyrocarbonic acid, having general formula R-O-CO-O-CO-O-R'. Preferably, R and R' are alkyl residues with between 1 and 3 carbon atoms. Preferably, R and R' are selected separately from an alkyl residue comprising 1, 2, and 3 carbon atoms.

An example of a pyrocarbonic acid ester according to the invention is diethyl pyrocarbonate or DEPC, with R and R' each being ethyl. The compound is also known as diethyl oxydiformate, ethoxyformic anhydride, or pyrocarbonic acid diethyl ester. Another example is dimethyl dicarbonate or DMDC, with R and R' each being methyl. Both, DEPC and DMDC are colourless liquids at room temperature.

In a preferred embodiment of the invention, the concentration of DEPC or DMDC in the composition according to the invention is between about 0.75% [v/v] and about 1.5% [v/v], more preferred between about 0.8% [v/v] and 1.2% [v/v], even more preferred between about 0.9% [v/v] and about 1.1% [v/v], and most preferred about 1% [v/v]. Further preferred, the concentration of DEPC or DMDC in the composition according to the invention is between about 0.1 M and about 1 M, more preferred between about 0.5 M and about 0.8 M, and most preferred about 0.7 M.

The iodoacetate anion is an alkylating agent which acts on cysteine residues in proteins. It can be used to modify SH-groups to prevent the re-formation of disulfide bonds after the reduction of cystine residues to cysteine.

Maleimide is a chemical compound with the formula H₂C₂(CO)₂N-R". The double bond of maleimide reacts with a thiol group of cysteine to form a stable carbon-sulfur bond. Maleimide and its derivatives are prepared by reacting maleic anhydride with amines followed by dehydration. In maleimide R" is H, that is to say maleimide is of the formula H₂C₂(CO)₂NH. In a maleimide derivative according to the invention, the substituent in said N-R" group is a C1 to C3 alkyl group. Very much preferred, R" is methyl. However, maleimide in which R" is H is most preferred.

In a preferred embodiment of the invention, the concentration of the compound capable of modifying thiol groups in the composition according to the invention is between about 0.1 M and about 1 M, more preferred between about 0.5 M and about 0.8 M, and most preferred about 0.7 M.

When reacting the polypeptide amino groups with a modifying compound, the pH is preferably kept in a non-acidic range, more preferred at neutral or weakly alkaline pH, in order to minimize degrading effects of alkaline pH on RNA. However, modification of thiol groups can be done at neutral and weakly acidic pH values of which a preferred range includes about pH 6 to about pH 7, and about pH 6.5 more preferred.

In order to reduce or inactivate RNase activity, the composition according to the invention is incubated for between about 10 seconds to about 10 minutes. The time may be chosen according to the temperature at which the incubation takes place. A preferred temperature range is between about 0°C and about 37°C, more preferred between about 15°C and about 30°C, even more preferred between 18°C and 23°C.

A further aspect of the invention is a method to reduce RNase activity in a liquid sample comprising RNA and protein with RNase activity, comprising the step of forming a composition according to the invention, and incubating said composition. The RNase activity in the sample (the lysate thereof) is thereby reduced by more than about 50%, preferably more than about 70%, even more preferred by more than about 80%, even more preferred by more than about 90%, even more preferred by about 95%, even more preferred by about 100%.

Compositina and methods according to the invention may form part of protocols for sample preparation which include the isolation of purified RNA. There is a large number of protocols known to the art concerning RNA purification.

Thus, a further aspect of the invention is a method to isolate RNA from a liquid sample comprising RNA and protein with RNase activity, comprising the steps of (a) reducing RNase activity in said sample by applying the method according to the invention, whereby a composition with reduced RNase activity is provided; followed by (b) purifying the RNA from said composition. Methods for purification of nucleic acids including RNA can be broadly classified within three categories: differential solubility, adsorption chromatography, and centrifugation methods. Differential solubility makes use of extraction in organic solvents such as phenol and chloroform. Adsorption chromatography binds nucleic acids to a matrix in the presence of chaotropic agents. Centrifugation methods include differential centrifugation and density gradient centrifugation. Very much preferred are procedures in which RNA is adsorbed from an adsorption solution to a solid phase, followed by separating the solid phase from the adsorption solution, and eluting the RNA from the solid phase. In order to facilitate binding of RNA to the solid phase, the adsorption solution is conditioned with compounds such as chaotropic agents, preferably a guanidine salt, and/or organic solvents of which C1-C5 alcohols are preferred.

Having in mind RNA purification using adsorption to a solid phase, the inventors also contemplate kits which provide materials and reagents for carrying out RNA purification. Thus, another aspect of thei invention is a kit of parts, comprising in separate containers (i) a compound capable of reacting in aqueous solution with a functional group of an amino acid residue in a polypeptide, whereby the functional group is selected from the group consisting of a primary amino group and a thiol group; (ii) a solid phase capable of reversibly binding RNA; (iii) a reagent for effecting lysis of biological material. Magnetic or paramagnetic particles with a surface comprising silica are a solid phase which is very much preferred. However, another preferred are solid phase is a fleece or filter material comprising silica fibers.

Using methods and kits according to the invention, yet another aspect of the invention is a method for determining the presence of a target RNA molecule in a liquid sample comprising RNA and protein with RNase activity, comprising the steps of (a) isolating RNA from the sample according to the invention; followed by (b) detecting in the isolated RNA the presence of the target RNA molecule. A preferred method includes (i) reverse transcribing the RNA to form a cDNA; (ii) amplifying, by means of the polymerase chain reaction, the cDNA or a fragment thereof; (iii) detecting the presence of the cDNA or the fragment thereof, thereby determining the presence of the target RNA molecule. Real time PCR is a particular useful tool to detect and quantify target cDNA and highly preferred, according to the invention.

The description is provided to aid the understanding of the present invention, the true scope of which is set forth in the appended claims. It is understood that modifications can be made in the procedures set forth without departing from the spirit of the invention.

## Claims

1. A composition consisting of (i) a liquid sample comprising RNA and protein with RNase activity, and (ii) a compound capable of reacting in aqueous solution with a functional group of an amino acid residue in a polypeptide, whereby the functional group is selected from the group consisting of a primary amino group and a thiol group,
**characterized in that** in the composition said compound is present at a concentration of between about 0.1 M and about 1 M.

2. The composition according to claim 1, **characterized in that** said compound is selected from the group consisting of a pyrocarbonic acid ester, a salt with an iodoacetate anion, and maleimide or a maleimide derivative.

3. The composition of claim 2, **characterized in that** said pyrocarbonic acid ester is diethyl pyrocarbonate or dimethyl dicarbonate.

4. The composition according to any of the claims 1 to 3, **characterized in that** said sample is a body fluid selected from the group consisting of whole blood, blood serum, blood plasma, cerebral fluid, sputum, stool suspension, a biopsy specimen, bone marrow, oral rinse, urine, and a lysate thereof.

5. The composition according to claim 4, **characterized in that** said sample is a lysate, and the sample further consists of one or more compounds selected from the group consisting of water, a buffer salt, an enzyme capable of degrading cellular walls, a proteolytic enzyme, a guanidine salt, and an anionic, cationic, zwitterionic or non-ionic detergent.

6. The composition according to claim 5, **characterized in that** said sample is a cleared lysate.

7. A method to reduce RNase activity in a liquid sample comprising RNA and protein with RNase activity, comprising the step of forming a composition according to any of the claims 1 to 6, and incubating said composition.

8. The method according to claim 4, **characterized in that** said composition is incubated at a temperature of between about 0°C and about 40°C.

9. The method according to claim 8, **characterized in that** after forming said composition is incubated for between about 10 seconds to about 10 minutes.

10. A method to isolate RNA from a liquid sample comprising RNA and protein with RNase activity, comprising the steps of (a) reducing RNase activity in said sample by applying the method according to any of the claims 7 to 9, whereby a composition with reduced RNase activity is provided; followed by (b) purifying the RNA from said composition.

11. The method according to claim 10, **characterized in that** step (b) comprises adsorbing the RNA to a solid phase, separating the solid phase from the composition, and eluting the RNA from the solid phase.

12. A method for determining the presence of a target RNA molecule in a liquid sample comprising RNA and protein with RNase activity, the method comprising the steps of (a) isolating RNA from the sample according to any of the claims 10 and 11; followed by (b) detecting in the isolated RNA the presence of the target RNA molecule.

13. The method according to claim 12, **characterized in that** step (b) comprises (i) reverse transcribing the RNA to form a cDNA; (ii) amplifying, by means of the polymerase chain reaction, the cDNA or a fragment thereof; (iii) detecting the presence of the cDNA or the fragment thereof, thereby determining the presence of the target RNA molecule.

14. The method according to claim 13, **characterized in that** step (iii) is performed by real-time PCR.

15. A kit of parts, comprising in separate containers (i) a compound capable of reacting in aqueous solution with a functional group of an amino acid residue in a polypeptide, whereby the functional group is selected from the group consisting of a primary amino group and a thiol group; (ii) a solid phase capable of reversibly binding RNA; (iii) a reagent for effecting lysis of biological material.
